(19) Europäisches Patentamt
European Patent Office
Office européen des brevets

(11) Numéro de publication: **0 410 834 A1**

(12) **DEMANDE DE BREVET EUROPEEN**

(21) Numéro de dépôt: **90401975.9**

(22) Date de dépôt: **09.07.90**

(51) Int. Cl.[5]: **C07D 273/04**, C07D 413/04, C07D 253/10, C07D 285/16, A61K 31/53, A61K 31/535, A61K 31/54

(30) Priorité: **17.07.89 FR 8909590**

(43) Date de publication de la demande: **30.01.91 Bulletin 91/05**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Malleron, Jean-Luc**
**2 allée Renoir**
**F-91 460 Marcoussis(FR)**

Inventeur: **Mansuy, Daniel**
**192 A rue de Vaugirard**
**F-75 015 Paris(FR)**
Inventeur: **N'Guyen, Thi-Vuong**
**24 rue des Trois-Bornes**
**F-75 011 Paris(FR)**
Inventeur: **Varech, Daniel**
**11 Rue Saint Louis en l'Ile**
**F-75 004 Paris(FR)**

(74) Mandataire: **Savina, Jacques et al**
**RHONE-POULENC SANTE, Service Brevets,**
**20 Avenue Raymond Aron**
**F-92165 Antony Cédex(FR)**

(54) **Médicaments à base de dérivés de benzoxadiazine-4, 1, 2 nouveaux dérivés et leurs procédés de préparation.**

(57) Médicaments contenant en tant que principe actif au moins un composé de formule :

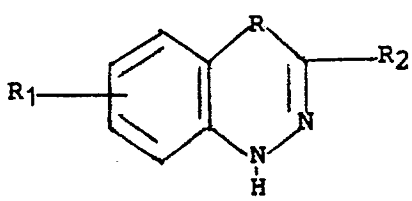

(I)

- R représente un atome d'oxygène, de soufre ou d'azote substitué par un radical alkyle,
- $R_1$ représente un atome d'hydrogène ou d'halogène ou un radical alkyle,
- $R_2$ représente un radical phényle, phényle substitué (par un radical alcoxy ou hydroxy), pyridyle, thiényle, naphtyle ou pentafluoro-2,3,4,5,6 phényle

les composés de formule (I) nouveaux et leurs procédés de préparation.

EP 0 410 834 A1

# MEDICAMENTS A BASE DE DERIVES DE 1H-BENZOXADIAZINE-4,1,2 NOUVEAUX DERIVES ET LEURS PROCEDES DE PREPARATION

La présente invention concerne des médicaments contenant en tant que principe actif au moins un dérivé de formule :

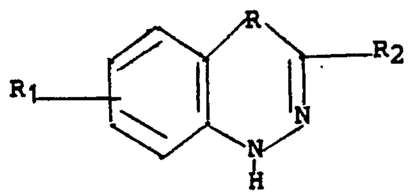

(I)

les nouveaux composés de formule (I) et leurs procédés de préparation.

Dans la formule (I),

- R représente un atome d'oxygène, de soufre ou d'azote substitué par un radical alkyle,
- $R_1$ représente un atome d'hydrogène ou d'halogène ou un radical alkyle et
- $R_2$ représente un radical phényle, phényle substitué (par un radical alcoxy ou hydroxy), pyridyle, thiényle, naphtyle ou pentafluoro-2,3,4,5,6 phényle, étant entendu que les radicaux alkyle et alcoxy contiennent 1 à 4 atomes de carbone en chaîne droite ou ramifiée.

La bromo-6 phényl-3 1H-benzoxadiazine-4,1,2, les bromo-6, -7, ou 8- ou fluoro-6 phényl-3 1H-benzothiadiazine-4,1,2, la bromo-6 p-méthoxyphényl-3 1H-benzothiadiazine-4, 1, 2 et la phényl-3 1H-benzothiadiazine-4, 1, 2 sont décrits dans la littérature mais aucune propriété pharmacologique n'a été mentionnée pour ces produits (A. J. ELLIOT et coll., J. Chem. Soc. Perkin trans., I, 23, 2915(1972), I.T. BARNISH et coll., J. Chem. Soc(C), 854(1970)) ; D. J. VUKOV et coll., J. Chem. Soc. Perkin Trans, I, 192 (1977) ; P. D. CALLAGHAN et coll., J. Chem. Soc. Perkin Trans, I, 1386 (1975)) et I. T. BARNISH et coll., J. Chem. Soc, Perkin Trans I, 215, (1974)).

La présente invention concerne également les composés de, formule (I) qui sont nouveaux.

Les composés de formule (I) pour lesquels R représente un atome d'oxygène ou de soufre peuvent être préparés par désacétylation d'un dérivé de formule :

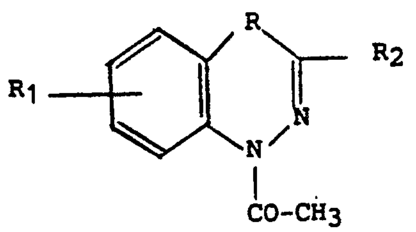

(II)

dans laquelle $R_1$ et $R_2$ ont les mêmes significations que dans la formule (I) et R représente un atome d'oxygène ou de soufre.

La désacétylation peut s'effectuer par toutes les méthodes connues de l'homme de l'art pour désacétyler une amine sans modifier le reste de la molécule. On peut, par exemple, utiliser les méthodes décrites dans Protective groups in organic synthesis, T.W. GREENE, p 252(1981).

De préférence, la désacétylation est effectuée au moyen d'une base telle que l'hydrazine hydrate à une température comprise entre 20 °C et la température d'ébullition du milieu réactionnel, la soude ou la potasse, au sein d'un solvant inerte tel que le diméthylformamide à la température d'ébullition du solvant ou un acide tel que l'acide sulfurique à une température voisine de 20 °C.

Les dérivés acétylés de formule (II) pour lequels R représente un atome d'oxygène peuvent être obtenus par cyclisation d'un dérivé de formule :

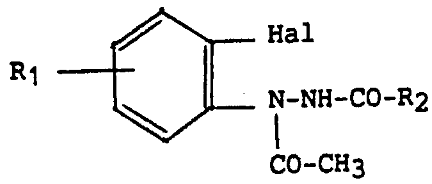

(III)

dans laquelle $R_1$ et $R_2$ ont les mêmes significations que dans la formule (I) et Hal représente un atome d'halogène (brome ou fluor de préférence).

Cette cyclisation s'effectue généralement au moyen d'une base telle que la soude ou la potasse, en présence d'une amine tertiaire telle que la triéthylamine, au sein d'un solvant inerte tel que le diméthylformamide, à une température comprise entre 20° C et la temperature d'ébullition du solvant.

Les composés de formule (III) peuvent être obtenus par action d'un dérivé de formule :

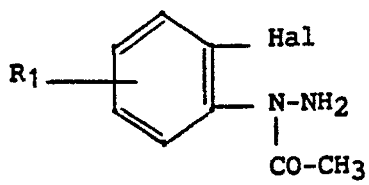

(IV)

dans laquelle $R_1$ et Hal ont les mêmes significations que dans la formule (III), sur un dérivé de formule :
Hal′-CO-$R_2$ (V)
dans laquelle Hal′ représente un atome d'halogène et $R_2$ a les mêmes significations que dans la formule (I).

Cette réaction s'effectue selon des procédés connus en soi tels que ceux décrits dans MARCH, Advanced Organic Chemistry, p 370(1985) Wiley Interscience.

De préférence, on opère au sein d'un solvant inerte tel qu'un solvant chloré (chloroforme, dichlorométhane par exemple) en présence d'une amine tertiaire telle que la triéthylamine, à une température comprise entre 0° C et 25° C.

Les dérivés de formule (IV) peuvent être obtenus par action d'un dérivé de formule :

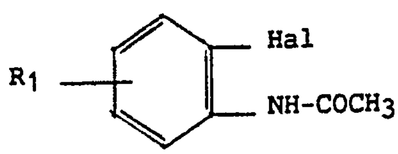

(VI)

dans laquelle $R_1$ et Hal ont les mêmes significations que dans la formule (III), sur l'o-diphénylphosphinylhydroxylamine.

Cette réaction s'effectue généralement en présence d'un hydrure de métal alcalin, au sein d'un solvant inerte tel que le diméthylformamide ou le tétrahydrofuranne, à une température voisine de 20° C.

L'o-diphénylphosphinylhydroxylamine peut-être préparée selon la méthode décrite par W. KLOTZER, Org. Synth., 64, 96(1986).

Les dérivés de formule (VI) peuvent être obtenus par acétylation des amines correspondantes selon des procédés connus en soi tels que ceux décrits dans MARCH, Advanced Organic Chemistry, p 370(1985) Wiley Interscience ou par application ou adaptation des méthodes décrites par B.P. GUPTA et coll., Labdev, part A, 10(3-4), 149(1972), Chem. Abst 80, 3218 T. DOORNBOS, Org. Prep. Proced., 1(4), 287-(1969) S.A. BENEZRA et coll., Z. Naturforsch., 274), 670(1972).

Les dérivés de formule (III) peuvent également être obtenus par action d'acétate de sodium sur un dérivé de formule :

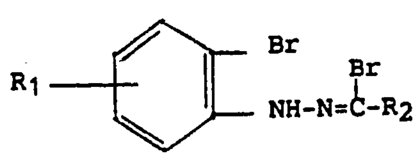

(VII)

dans laquelle $R_1$ et $R_2$ ont les mêmes significations que dans la formule (I).

Cette réaction s'effectue de préférence au sein de l'acide acétique, à une température comprise entre 20° C et la température d'ébullition du solvant.

Les dérivés de formule (VII) peuvent être obtenus par bromuration d'un dérivé de formule :

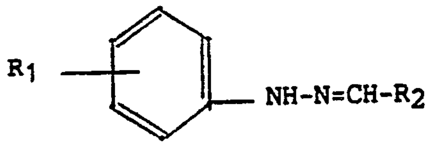

(VIII)

dans laquelle $R_1$ et $R_2$ ont les mêmes significations que dans la formule (I).

Cette bromuration s'effectue généralement au moyen de brome, au sein de l'acide acétique, à une température comprise entre 10° C et la température d'ébullition du solvant.

Les dérivés de formule (VIII) peuvent être obtenus par application ou adaptation des méthodes décrites par B.A. DELLACOLETTA et coll., J. Org. Chem., 3057(1977) et dans le brevet DE 2 744 385.

De préférence, on fait réagir un dérivé de formule :

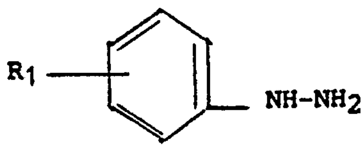

(IX)

dans laquelle $R_1$ a les mêmes significations que dans la formule (I) sur un aldéhyde de formule :

$R_2$-CHO (X)

dans laquelle $R_2$ a les mêmes significations que dans la formule (I).

Cette réaction s'effectue généralement au sein d'un solvant chloré, en présence d'une amine tertiaire telle que la triéthylamine, à une température comprise entre 0° C et la température d'ébullition du solvant.

Les dérivés de formule (III) à l'exception de ceux pour lesquels $R_2$ représente un radical hydroxyphényle peuvent également être obtenus par acétylation des dérivés de formule :

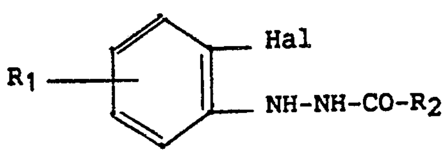

(XI)

dans laquelle $R_1$ et $R_2$ ont les mêmes significations que dans la formule (I) à l'exception que $R_2$ ne peut pas représenter un radical hydroxyphényle et Hal représente un atome d'halogène (brome ou fluor de préférence).

Cette réaction s'effectue dans les mêmes conditions que celles mentionnées précédemment pour la préparation des dérivés de formule (VI).

Les dérivés de formule (XI) peuvent être obtenus par action d'un dérivé de formule :

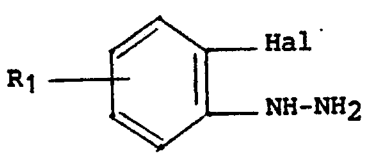

(XII)

dans laquelle $R_1$ et Hal ont les mêmes significations que dans la formule (XI) sur un dérivé de formule (V).

Cette réaction s'effectue généralement dans les conditions décrites précédemment pour la préparation des composés de formule (III) par action des dérivés de formules (IV) et (V).

Les dérivés de formule (II) pour lesquels $R_1$ représente un atome d'iode et R représente un atome d'oxygène peuvent être obtenus par action d'un nitrite de métal alcalin puis d'iodure de potassium sur un dérivé aminé de formule :

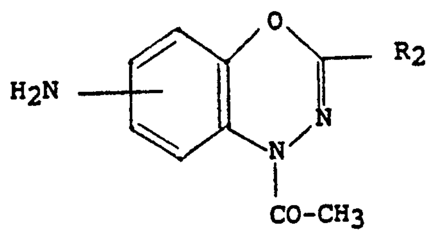

(XIII)

dans laquelle $R_2$ a les mêmes significations que dans la formule (II).

Cette réaction s'effectue par toutes les méthodes connues de l'homme de l'art pour transformer une amine en un atome d'iode telles que celles décrites dans MARCH, Advanced Organic Chemistry, p 602-(1985) Wiley Interscience.

De préférence on fait réagir le nitrite de sodium sur le dérivé aminé en milieu acide à une température voisine de 5° C puis on fait régir in situ l'iodure de potassium, à une température voisine de 20° C.

Les dérivés aminés de formule (XIII) peuvent être obtenus par réduction des dérivés nitrés correspondants par toutes les méthodes connues de l'homme de l'art pour transformer une fonction nitro en une fonction amine telles que celles décrites dans MARCH, Advanced Organic Chemistry p 1103(1985) Wiley Interscience .

Les dérivés nitrés peuvent être obtenus par les méthodes décrites précédemment pour les composés de formule (II).

Les dérivés de formule (II) pour lesquels R représente un atome de soufre peuvent être préparés par application ou adaptation de la méthode décrite par I.T. BARNISH et coll., J. Chem. Soc(C), 854(1970).

Les composés de formule (I) pour lesquels R représente un atome d'oxygène à l'exception de ceux pour lesquels $R_2$ représente un radical alcoxyphényle peuvent également être préparés par cyclisation d'un dérivé de formule :

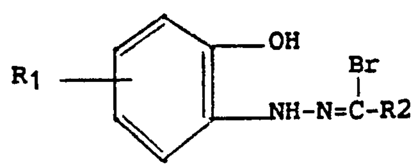

(XIV)

dans laquelle $R_1$ et $R_2$ ont les mêmes significations que dans la formule (I) à l'exception pour $R_2$ de représenter un radical alcoxyphényle.

Cette réaction s'effectue généralement au moyen d'un alcoolate de métal alcalin (méthylate de sodium ou de potassium, éthylate de sodium ou de potassium), au sein d'un alcool, à une température comprise entre -50° C et 30° C.

Les dérivés de formule (XIV) peuvent être obtenus par déméthylation d'un dérivé de formule :

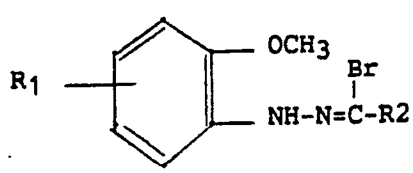

(XV)

dans laquelle $R_1$ et $R_2$ ont les mêmes significations que dans la formule (XIV).

Cette réaction s'effectue de préférence au moyen de tribromure de bore, au sein d'un solvant inerte tel qu'un solvant chloré (chloroforme, dichlorométhane...), à une température comprise entre 0° C et 20° C.

Les dérivés de formule (XV) peuvent être obtenus par bromuration d'un dérivé de formule :

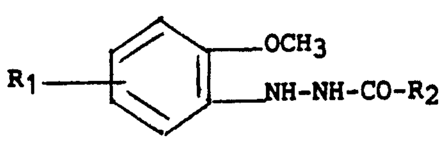

(XVI)

dans laquelle $R_1$ et $R_2$ ont les mêmes significations que dans la formule (XIV).

Cette réaction s'effectue généralement au moyen soit de tétrabromure de carbone en présence de triphénylphosphine au sein d'un solvant inerte tel que le tétrahydrofuranne ou l'acétonitrile, à une température voisine de 20 °C.

Les dérivés de formule (XVI) peuvent être obtenus par action d'un halogène de formule :

$Hal\text{-}CO\text{-}R_2$ (XVII)

dans laquelle Hal représente un atome d'halogène (chlore ou brome de préférence) et $R_2$ a les mêmes significations que dans la formule (XIV), sur un dérivé de formule :

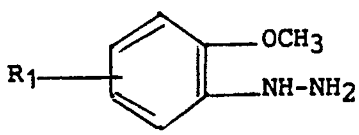

(XVIII)

dans laquelle $R_1$ a les mêmes significations que dans la formule (I).

Cette réaction s'effectue au sein d'un solvant inerte tel qu'un éther (éther diéthylique par exemple) en présence de pyridine, à une température voisine de 20 °C.

Les composés de formule (XV) pour lesquels $R_1$ est en position-4 et représente un atome de brome peuvent également être obtenus par dibromuration d'un dérivé de formule :

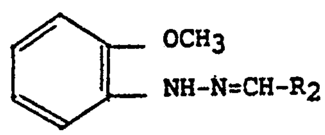

(XIX)

dans laquelle $R_2$ a les mêmes significations que dans la formule (XIV).

Cette dibromuration s'effectue généralement au moyen de tribromure de phényltriméthylammonium au sein d'un solvant inerte tel que le tétrahydrofuranne à une température voisine de 20 °C.

Les dérivés de formule (XIX) peuvent être obtenus par action d'un dérivé de formule (X) dans laquelle $R_2$ a les mêmes significations que dans la formule (XIV) sur un dérivé de formule (XVIII) dans laquelle $R_1$ représente un atome d'hydrogène.

Cette réaction s'effectue au sein d'un solvant inerte tel qu'un alcool, une cétone ou un éther, à une température voisine de 20 °C, éventuellement en présence d'une base telle que la triéthylamine ou la pyridine.

Les composés de formule (I) pour lesquels R représente un atome d'azote substitué par un radical alkyle à l'exception de ceux pour lesquels $R_2$ représente un radical hydroxyphényle peuvent être préparés par cyclisation d'un dérivé de formule :

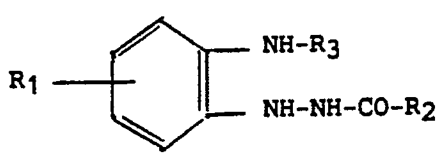

(XX)

dans laquelle $R_1$ et $R_2$ ont les mêmes significations que dans la formule (I) excepté que $R_2$ ne peut pas représenter un radical hydroxyphényle et $R_3$ représente un radical alkyle.

Cette réaction s'effectue généralement en présence de tétrabromure de carbone et de triphénylphosphine au sein d'un solvant inerte tel que l'acétonitrile, à une température voisine de 20 °C.

Les dérivés de formule (XX) peuvent être obtenus par action d'un halogènure d'alkyle sur un dérivé de formule :

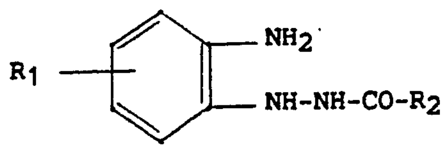

(XXI)

dans laquelle $R_1$ et $R_2$ ont les mêmes significations que dans la formule (XX).

Cette réaction s'effectue généralement au sein d'un solvant inerte tel que l'hexaméthylphosphortriamide, en présence d'hydrogénocarbonate de sodium, à une température voisine de 20 °C.

Les dérivés de formule (XXI) peuvent être obtenus par application ou adaptation de la méthode décrite par M.N. SHENG et coll., J. Org. Chem., 28, 736(1963).

Les composés de formule (I) pour lesquels $R_2$ représente un radical hydroxyphényle peuvent être préparés par déméthylation des dérivés de formule (I) correspondants pour lesquels $R_2$ représente un radical alcoxyphényle.

Cette réaction s'effectue généralement par toutes les méthodes connues de l'homme de l'art pour transformer un radical alcoxy en un radical hydroxy sans toucher au reste de la molécule.

On opère de préférence au moyen de tribromure de bore, au sein d'un solvant chloré (chlorure de méthylène, chloroforme, dichlorométhane par exemple), à une température voisine de 20 °C.

Les mélanges réactionnels obtenus par les divers procédés décrits précédemment sont traités suivant des méthodes classiques physiques ou chimiques (évaporation, extraction, distillation, cristallisation, chromatographie...).

Les composés de formule (I), présentent des propriétés pharmacologiques intéressantes dans le domaine de l'inflammation. Ces composés sont des inhibiteurs de 5-lipoxygènase et de cyclooxygènase et sont donc utiles comme antiinflammatoires pour le traitement de l'asthme, des maladies allergiques, du psoriasis ainsi que pour le traitement de tout phénomène pathologique cutané lié à l'hyperactivité de la 5-lipoxygènase et de la cyclooxygènase.

L'inhibition de la 5-lipoxygènase et de la cyclooxygènase a été déterminée sur des cellules RBL-1 selon les méthodes de M.M. STEINHOFF et coll., B.B.A., 618, 28-34(1980) et B.A. JACKSCHIK et coll., J. Biol. Chem., 257, 5346(1982). Dans ce test la $CI_{50}$ des composés de formule (I) est inférieure à $10^{-5}$M sur la 5-lipoxygènase et inférieure à 5 $10^{-5}$M sur la cyclooxygènase.

Les composés de formule (I) se sont également montrés actifs par voie topique sur l'oedème de l'oreille de la souris déclenché à l'acide arachidonique dans les conditions suivantes : 30 minutes avant le déclenchement de l'oedème, 20 mm3 d'une solution acétonique contenant de 0,01 à 1 mg d'un produit de formule (I), sont déposés sur la face interne de l'oreille droite de souris mâles OF1 et 20 mm3 d'acétone sont déposés sur l'oreille gauche servant de contrôle. L'oedème est déclenché par dépôt de 20 mm3 d'une solution acétonique contenant 1 mg d'acide arachidonique, sur la face interne de l'oreille droite. Une heure après ce traitement, les animaux sont sacrifiés par élongation cervicale et les oreilles sont coupées à leur base et pesées. Le pourcentage d'inhibition est déterminé par mesure de la différence de poids des oreilles droite et gauche et comparaison aux souris témoins n'ayant pas reçu de produit de formule (I).

Dans ce test la $DE_{50}$ des composés de formule (I) est inférieure à 0,5mg/oreille.

Les composés de formule (I) présentent une faible toxicité. Leur $DL_{50}$ est généralement supérieure à 100mg/kg chez la souris par voie intra-péritonéale.

Les exemples suivants montrent comment l'invention peut être mise en pratique.

EXEMPLE 1

79g d'acétyl-1 (dibromo-2,4 phényl)-1 benzoyl hydrazine, 18,7g de potasse en pastilles et 96 cm3 de triéthylamine dans 600 cm3 de diméthylformamide sont agités et chauffés à ébullition pendant 6 heures. Le mélange est refroidi à une température voisine de 20 °C puis concentré à sec à 70 °C sous pression réduite (0,05 mm de mercure ; 0,07 kPa). L'huile résiduelle est reprise par 500 cm3 d'eau distillée et par 500 cm3 de dichlorométhane. La phase organique est décantée, séchée sur du sulfate de sodium anhydre, filtrée et concentrée à sec à 40 °C sous pression réduite (20 mm de mercure ; 2,7 kPa). Le résidu solide est repris par 650 cm3 d'hydrazine hydrate ; le mélange est chauffé à une température proche de 60 °C pendant 15 heures puis refroidi à une température voisine de 20 °C. Le précipité est filtré, lavé par 2 fois 200 cm3 d'eau distillée puis repris par 1500 cm3 de dichlorométhane et 400 cm3 d'eau distillée. La phase organique est décantée, séchée sur du sulfate de magnésium anhydre, filtrée et concentrée à sec à 40 °C

sous pression réduite (20 mm de mercure ; 2,7 kPa). Le solide résiduel est purifié par recristallisation dans 140 cm3 de toluène bouillant. On obtient 37,8g de bromo-6 phényl-3 1H-benzoxadiazine-4,1,2 fondant à 154 °C.

L'acétyl-1 (dibromo-2,4 phényl)-1 benzoyl-2 hydrazine peut être préparée de la manière suivante : 100 g de bromure de N-(dibromo-2,4 phényl) benzènecarbohydrazonoyle et 95g d'acétate de sodium dans 1000 cm3 d'acide acétique sont agités et chauffés à ébullition 4 heures. Le mélange est refroidi à une température voisine de 20 °C puis concentré à sec à 40 °C sous pression réduite (20 mm de mercure ; 2,7 kPa). L'huile résiduelle est reprise par 800 cm3 d'eau distillée et 800 cm3 de dichlorométhane. La phase organique est décantée, lavée par 2 fois 1000 cm3 d'eau distillée, séchée sur du sulfate de sodium anhydre et concentrée à sec à 40 °C sous pression réduite (20 mm de mercure ; 2,7 kPa). Le résidu solide est purifié par recristallisation dans 400 cm3 d'isopropanol bouillant. On obtient 79g d'acétyl-1 (dibromo-2,4 phényl)-1 benzoyl-2 hydrazine fondant à 158 °C, utilisée à l'état brut dans les synthèses ultérieures.

Le bromure de N-(dibromo-2,4 phényl) benzènecarbohydrazonoyle peut être préparé de la façon suivante : à 98g de benzaldéhyde phényl-hydrazone dans 2500 cm3 d'acide acétique, on coule en 20 minutes, à une température proche de 20 °C et en maintenant l'agitation, 77 cm3 de brome. Le mélange est agité 3 heures à une température voisine de 20 °C puis repris par 1500 cm3 d'eau distillée. Le précipité formé est filtré sur verre fritté, lavé par 2 fois 500 cm3 d'eau distillée et dissous dans 1500 cm3 de dichlorométhane. La phase organique est reprise par 500 cm3 d'eau distillée, décantée, séchée sur du sulfate de magnésium anhydre, filtrée et concentrée à sec à 40 °C sous pression réduite (20 mm de mercure ; 2,7 kPa). On obtient 200g de bromure de N-(bromo-2,4 phényl) benzènecarbohydrazonoyle fondant à 111 °C, utilisé à l'état brut dans les synthèses ultérieures.

## EXEMPLE 2

3,8g d'acétyl-1 (pyridyl-3)-3 1H-benzoxadiazine-4,1,2 et 38 cm3 d'hydrazine hydrate sont agités et chauffés à une température proche de 50 °C pendant 2 heures. Le mélange est refroidi à une température voisine de 20 °C et repris par 100 cm3 d'eau distillée. Le solide est filtré sur verre fritté et lavé par 2 fois 30 cm3 d'eau distillée. Il est purifié par recristallisation dans 200 cm3 de cyclohexane bouillant. On obtient 1,3g de (pyridyl-3)-3 1H-benzoxadiazine-4,1,2 fondant à 135 °C.

L'acétyl-1 (pyridyl-3)-3 1H-benzoxadiazine-4,1,2 peut être préparée de la façon suivante : 12,7g d'acétyl-1 (bromo-2 phényl)-1 nicotinoyl-2 hydrazine, 3,2g de potasse en pastilles et 19 cm3 de triéthylamine dans 200 cm3 de diméthylformamide sont agités et chauffés au reflux pendant 6 heures. Le mélange est refroidi à une température proche de 20 °C et repris par 1000 cm3 d'eau distillée. La phase organique est extraite par 2 fois 200 cm3 de dichlorométhane, lavée par 300 cm3 d'eau distillée, séchée sur du sulfate de sodium anhydre, filtrée et concentrée à sec à 40 °C sous pression réduite (20 mm de mercure ; 2,7 kPa). L'huile résiduelle est purifiée par flash chromatographie sur colonne de silice, sous courant d'argon, à moyenne pression (0,5-1,5 bar) avec de l'acétate d'éthyle comme éluant. On obtient 3,8g d'acétyl-1 pyridyl-3 1H-benzoxadiazine-4,1,2 fondant à 80 °C, utilisée à l'état brut dans les synthèses ultérieures.

L'acétyl-1 (bromo-2 phényl)-1 nicotinoyl-2 hydrazine peut être préparée de la manière suivante : à une solution de 11,5g d'acétyl-1 (bromo-2 phényl)-1 hydrazine et de 14 cm3 de triéthylamine dans 115 cm3 de dichlorométhane refroidie à 0 °C, on ajoute 8,9g de chlorhydrate de chlorure de nicotinoyle. Le mélange est agité une heure à une température proche de 20 °C puis est repris par 100 cm3 d'eau distillée. Le précipité formé est filtré sur verre fritté et lavé par 2 fois 50 cm3 de dichlorométhane. On obtient ainsi 9,2g d'acétyl-1 (bromo-2 phényl)-1 nicotinoyl-2 hydrazine fondant à 210 °C et utilisée à l'état brut dans les synthèses ultérieures.

L'acétyl-1 (bromo-2 phényl)-1 hydrazine peut être préparée de la manière suivante : à un mélange de 12g d'hydrure de sodium en dispersion à 50% dans l'huile de vaseline dans 700 cm3 de diméthylformamide, sous courant d'argon, on ajoute en 15 minutes, à une température voisine de 20 °C, 42,8g de N-acétyl bromo-2 aniline dans 100 cm3 de diméthylformamide. L'agitation est maintenue 15 minutes à cette température puis le mélange réactionnel est refroidi à une température proche de 10 °C. On ajoute en 30 minutes, par portion, 58 g d'O-diphénylphosphinylhydroxylamine dans 300 cm3 de diméthylformamide et maintient l'agitation 2 heures à une température voisine de 20 °C. Le précipité formé est filtré sur verre fritté et lavé par 3 fois 200 cm3 de diméthylformamide. Le filtrat est concentré à sec à 70 °C sous pression réduite (0,5 mm de mercure ; 0,07 kPa). L'huile résiduelle est purifiée par flash-chromatographie sur colonne de silice, sous courant d'argon, à moyenne pression (0,5-1,5 bar) avec un mélange de cyclohexane et d'acétate d'éthyle(30-70 en volumes) comme éluant. On obtient 35,2g d'acétyl-1 (bromo-2 phényl)-1 hydrazine fondant à 75 °C et utilisée à l'état brut dans les synthèses ultérieures.

La N-acétyl bromo-2 aniline peut être préparée selon la méthode décrite par B.P. GUPTA et coll., Labdev, part A, 10(3-4), 149(1972) (C.A.80,3218).

L'O-diphénylphosphinylhydroxylamine peut être préparée selon la méthode décrite par W. KLOTZER, Org. synth., 64,96(1986).

EXEMPLE 3

On opère comme à l'exemple 2, à partir de 3,2g d'acétyl-1 (thiényl-2)-3 1H-benzoxadiazine-4,1,2 et de 32 cm3 d'hydrazine hydrate. Le mélange est agité pendant 3 heures à une température proche de 80° C puis refroidi à une température voisine de 20° C. Après purification par recristallisation dans 160 cm3 de cyclohexane, on obtient 2,1g de (thiényl-2)-3 1H-benzoxadiazine-4,1,2 fondant à 136° C.

L'acétyl-1 (thiényl-2)-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 13,7g d'acétyl-1 (bromo-2 phényl)-1 (thénoyl-2)-2 hydrazine, de 20 cm3 de triéthylamine et de 3,4g de potasse en pastilles dans 200 cm3 de diméthylformamide. La solution est agitée pendant 6 heures à reflux puis refroidie à une température proche de 20° C. Après purification par flash-chromatographie sur colonne de silice, sous courant d'argon, à moyenne pression (0,5-1,5 bar), on obtient 3,2g d'acétyl-1 (thiényl-2)-3 1H-benzoxadiazine-4,1,2 fondant à 95° C, utilisée à l'état brut dans les synthèses ultérieures.

L'acétyl-1 (bromo-2 phényl)-1 (thénoyl-2)-2 hydrazine peut être préparée de la manière suivante : on opère comme à l'exemple 2 pour la préparation de l'acétyl-1 (bromo-2 phényl)-1 nicotinoyl-2 hydrazine, à partir de 11,5g d'acétyl-1 (bromo-2 phényl)-1 hydrazine, de 7,3g de chlorure de thénoyle-2 et de 7 cm3 de triéthylamine dans 115 cm3 de dichlorométhane. Le mélange est agité 2 heures à une température proche de 20° C. Après purification par recristallisation dans 170 cm3 d'isopropanol bouillant, on obtient 13,7g d'acétyl-1 (bromo-2 phényl)-1 (thénoyl-2)-2 hydrazine fondant à 160° C et utilisée à l'état brut dans les synthèses ultérieures.

EXEMPLE 4

On opère comme à l'exemple 2, à partir de 2,4g d'acétyl-1 (naphtyl-2)-3 1H-benzoxadiazine-4,1,2 et de 24 cm3 d'hydrazine hydrate. Le mélange est agité pendant 2 heures à une température proche de 80° C puis refroidi à une température voisine de 20° C. Après purification par recristallisation dans 58 cm3 d'isopropanol, on obtient 2,2g de (naphtyl-2)-3 1H-benzoxadiazine-4,1,2 fondant à 146° C.

L'acétyl-1 (naphtyl-2)-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 12,6g d'acétyl-1 (bromo-2 phényl)-1 (naphtoyl-2)-2 hydrazine, de 16,5 cm3 de triéthylamine et de 2,8g de potasse en pastilles dans 120 cm3 de diméthylformamide. La solution est agitée pendant 6 heures à reflux puis refroidie à une température proche de 20° C. Après purification par recristallisation dans 110 cm3 d'isopropanol bouillant, on obtient 2,4g d'acétyl-1 (naphtoyl-2)-3 1H-benzoxadiazine-4,1,2 fondant à 160° C, utilisée à l'état brut dans les synthèses ultérieures.

L'acétyl-1 (bromo-2 phényl)-1 (naphtoyl-2)-2 hydrazine peut être préparée de la manière suivante : on opère comme à l'exemple 2 pour la préparation de l'acétyl-1 (bromo-2 phényl)-1 nicotinoyl-2 hydrazine, à partir de 11,5g d'acétyl-1 (bromo-2 phényl)-1 hydrazine, de 9,7g de chlorure de naphtoyle-2 et de 7 cm3 de triéthylamine dans 115 cm3 de dichlorométhane. Le mélange est agité 2 heures à une température proche de 20° C. Après purification par recristallisation dans 100 cm3 d'isopropanol bouillant, on obtient 16,5g d'acétyl-1 (bromo-2 phényl)-1 (naphtoyl-2)-2 hydrazine fondant à 155° C et utilisée à l'état brut dans les synthèses ultérieures.

EXEMPLE 5

On opère comme à l'exemple 1, à partir de 7,1g d'acétyl-1 (dibromo-2,5 phényl)-1 benzoyl-2 hydrazine, 1,4g de soude en poudre et 8,6 cm3 de triéthylamine dans 100 cm3 de diméthylformamide. Le mélange est agité pendant 5 heures à reflux puis refroidi à une température voisine de 20° C. Après purification par flash-chromatographie sur colonne de silice, sous courant d'argon, à moyenne pression (0,5-1,5 bar) avec un mélange de dichlorométhane et de cyclohexane (50-50 en volumes) comme éluant et recristallisation dans 40 cm3 d'isopropanol bouillant, on obtient 1,2g de bromo-7 phényl-3 1H-benzoxadiazine-4,1,2 fondant

à 172° C.

L'acétyl-1 (dibromo-2,5 phényl)-1 benzoyl-2 hydrazine peut être préparée de la manière suivante : on opère comme à l'exemple 2 pour la préparation de l'acétyl-1 (bromo-2 phényl)-1 nicotinoyl-2 hydrazine, à partir de 10,3g d'acétyl-1 (dibromo-2,5 phényl)-1 hydrazine, de 4,7g de chlorure de benzoyle et de 4,7 cm3 de trié thylamine dans 100 cm3 de dichlorométhane. Le mélange est agité 2 heures à une température proche de 20° C. Après purification par recristallisation dans 270 cm3 d'isopropanol bouillant, on obtient 9,2g d'acétyl-1 (dibromo-2,5 phényl)-1 benzoyl-2 hydrazine fondant à 190° C et utilisée à l'état brut dans les synthèses ultérieures.

L'acétyl-1 (dibromo-2,5 phényl)-1 hydrazine peut être préparée de la manière suivante : on opère comme à l'exemple 2 pour la préparation de l'acétyl-1 (bromo-2 phényl)-1 hydrazine, à partir de 17,6g de N-acétyl dibromo-2,5 aniline, de 3g d'hydrure de sodium à 50% en dispersion dans l'huile de vaseline et de 14,5g d'O-diplénylphosphinylhydroxylamine dans 125 cm3 de diméthylformamide. Après purification par flash-chromatographie sur colonne de silice, sous courant d'argon, à moyenne pression (0,5-1,5 bar) avec un mélange d'acétate d'éthyle et de cyclohexane (70-30 en volumes) comme éluant, on obtient 10,5g d'acétyl-1 (dibromo-2,5 phényl)-1 hydrazine fondant à 114° C et utilisée à l'état brut dans les synthèses ultérieures.

La N-acétyl dibromo-2,5 aniline peut être préparée selon la méthode décrite par T. DOORNBOS, Org. Prep. Proced., 1(4), 287(1969).

EXEMPLE 6

On opère comme à l'exemple 2, à partir de 7,4g d'acétyl-1 bromo-8 phényl-3 1H-benzoxadiazine-4,1,2 et de 74 cm3 d'acide sulfurique 36N. Le mélange est agité pendant 1 heure à une température proche de 20° C. Après purification par recristallisation dans 105 cm3 d'éther de pétrole bouillant, on obtient 5,2g de bromo-8 phényl-3 1H-benzoxadiazine-4,1,2 fondant à 70° C.

L'acétyl-1 bromo-8 phényl-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 12,2g d'acétyl-1 (dibromo-2,6 phényl)-1 benzoyl-2 hydrazine, de 15 cm3 de triéthylamine et de 2,5g de potasse en pastilles dans 150 cm3 de diméthylformamide. La solution est agitée pendant 90 minutes à reflux puis refroidie à une température proche de 20° C. Le solide formé est filtré sur verre fritté et lavé par 2 fois 50 cm3 d'eau distillée ; on obtient 8,4g d'acétyl-1 bromo-8 phényl-3 1H-benzoxadiazine-4,1,2 fondant à 180° C, utilisée à l'état brut dans les synthèses ultérieures.

L'acétyl-1 (dibromo-2,6 phényl)-1 benzoyl-2 hydrazine peut être préparée de la manière suivante : on opere comme à l'exemple 2 pour la préparation de l'acétyl-1 (bromo-2 phényl)-1 nicotinoyl-2 hydrazine, à partir de 21,1g d'acétyl-1 (dibromo-2,6 phényl)-1 hydrazine, de 9,6g de chlorure de benzoyle et de 9,6 cm3 de triéthylamine dans 200 cm3 de dichlorométhane. Le mélange est agité 48 heures à une température proche de 20° C. Après purification par flash-chromatographie sur colonne de silice, sous courant d'argon, à moyenne pression (0,5-1,5 bars) avec un mélange d'acétate d'éthyle et de cyclohexane (20-80 en volumes) comme éluant, on obtient 13,5g d'acétyl-1 (dibromo-2,6 phényl)-1 benzoyl-2 hydrazine fondant à 150° C et utilisée à l'état brut dans les synthèses ultérieures.

L'acétyl-1 (dibromo-2,6 phényl)-1 hydrazine peut être préparée de la manière suivante : on opère comme à l'exemple 2 pour la préparation de l'acétyl-1 (bromo-2 phényl)-1 hydrazine, à partir de 24g de N-acétyl dibromo-2,6 aniline, de 4,1g d'hydrure de sodium à 50% en dispersion dans l'huile de vaseline et de 21,7g d'O-diphénylphosphinylhydroxylamine dans 200 cm3 de diméthylformamide. Le mélange est agité 1 heure à une température proche de 20° C. Après purification par cristallisation dans 150 cm3 d'éther de diisopropyle, on obtient 21,1g d'acétyl-1 (dibromo-2,6 phényl)-1 hydrazine fondant à 166° C et utilisée à l'état brut dans les synthèses ultérieures.

La N-acétyl dibromo-2,6 aniline peut être préparée selon la méthode décrite par S.A. BENEZRA et coll., Z. NATURFORSCH., 27(4), 670(1972).

EXEMPLE 7

On opère comme à l'exemple 2 ,à partir de 15g d'acétyl-1 chloro-6 phényl-3 1H-benzoxadiazine-4,1,2 et de 150 cm3 d'hydrazine hydrate. Le mélange est agité pendant 4 heures à une température proche de 70° C puis refroidi à une température voisine de 20° C. Après purification par recristallisation dans 400 cm3 de cyclohexane bouillant, on obtient 8,2g de chloro-6 phényl-3 1H-benzoxadiazine-4,1,2 fondant à 138° C.

L'acétyl-1 chloro-6 phényl-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 39g d'acétyl-1 (bromo-2 chloro-4 phényl)-1 benzoyl-2 hydrazine, de 50 cm3 de triéthylamine et de 4,3g de potasse en pastilles dans 250 cm3 de diméthylformamide. La solution est agitée pendant 6 heures à reflux puis refroidie à une température proche de 20 °C. Après purification par flash-chromatographie sur colonne de silice, sous courant d'argon, à moyenne pression (0,5-1,5 bar) avec du dichlorométhane comme éluant, on obtient 17,9g d'acétyl-1 chloro-6 phényl-3 1H-benzoxadiazine-4,1,2 fondant à 135 °C, utilisée à l'état brut dans les synthèses ultérieures.

L'acétyl-1 (bromo-2 chloro-4 phényl)-1 benzoyl-2 hydrazine peut être préparée de la manière suivante : on opère comme à l'exemple 1 pour la préparation de l'acétyl-1 (dibromo-2,4 phényl)-1 benzoyl-2 hydrazine, à partir de 66g de bromure de N-(bromo-2 chloro-4 phényl) benzènecarbohydrazonoyle et de 71g d'acétate de sodium dans 1200 cm3 d'acide acétique. Le mélange est agité pendant 5 heures à reflux puis refroidi à une température proche de 20 °C. Après traitement habituel, on obtient 39g d'acétyl-1 (bromo-2 chloro-4 phényl)-1 benzoyl-2 hydrazine fondant à 122 °C et utilisée à l'état brut dans les synthèses ultérieures.

Le bromure de N-(bromo-2 chloro-4 phényl) benzènecarbohydrazonoyle peut être préparé de la manière suivante : on opère comme à l'exemple 1 pour la préparation du bromure de N-(dibromo-2,4 phényl) benzènecarbohydrazonoyle, à partir de 40,2g de benzaldéhyde (chloro-4 phényl) hydrazone et de 18 cm3 de brome dans 700 cm3 d'acide acétique. Le mélange est agité 30 minutes à reflux puis refroidi à une température proche de 20 °C. Après traitement habituel, on obtient 66g de bromure de N-(bromo-2 chloro-4 phényl) benzènecarbohydrazonoyle fondant à 116 °C et utilisé à l'état brut dans les synthèses ultérieures.

La benzaldéhyde (chloro-4 phényl) hydrazone peut être préparée selon la méthode décrite dans le brevet DE 2744385.

EXEMPLE 8

On opère comme à l'exemple 2, à partir de 1,5g d'acétyl-1 fluoro-6 phényl-3 1H-benzoxadiazine-4,1,2 et de 15 cm3 d'hydrazine hydrate. Le mélange est agité pendant 8 heures à une température proche de 70 °C puis refroidi à une température voisine de 20 °C. Après purification par flash-chromatographie sur colonne de silice, sous courant d'argon, à moyenne pression (0,5-1,5 bar) avec du dichlorométhane comme éluant et recristallisation dans 45 cm3 de cyclohexane bouillant, on obtient 0,9g de fluoro-6 phényl-3 1H-benzoxadiazine-4,1,2 fondant à 99 °C.

L'acétyl-1 fluoro-6 phényl-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 11,6 g d'acétyl-1 (bromo-2 fluoro-4 phényl)-1 benzoyl-2 hydrazine, de 16,5 cm3 de triéthylamine et de 1,3 g de soude en pastilles dans 83 cm3 de diméthylformamide. La solution est agitée pendant 6 heures à reflux puis refroidie à une température proche de 20 °C. Après purification par flash-chromatographie sur colonne de silice, sous courant d'argon, à moyenne pression (0,5-1,5 bar) avec du dichlorométhane comme éluant, on obtient 2,8g d'acétyl-1 fluoro-6 phényl-3 1H-benzoxadiazine-4,1,2 fondant à 129 °C, utilisée à l'état brut dans les synthèses ultérieures.

L'acétyl-1 (bromo-2 fluoro-4 phényl)-1 benzoyl-2 hydrazine peut être préparée de la manière suivante : on opère comme à l'exemple 1 pour la préparation de l'acétyl-1 (dibromo-2,4 phényl)-1 benzoyl-2 hydrazine, à partir de 31,3g de bromure de N-(bromo-2 fluoro-4 phényl) benzènecarbohydrazonoyle et de 38g d'acétate de sodium dans 500 cm3 d'acide acétique. Le mélange est agité pendant 5 heures à reflux puis refroidi à une température proche de 20 °C. Après purification par flash-chromatographie sur colonne de silice, sous courant d'argon, à moyenne pression (0,5-1,5 bar) avec un mélange d'acétate d'éthyle et de cyclohexane (70-30 en volumes) comme éluant, on obtient 15,1g d'acétyl-1 (bromo-2 fluoro-4 phényl)-1 benzoyl-2 hydrazine sous forme d'une huile brune et utilisée à l'état brut dans les synthèses ultérieures.

Le bromure de N-(bromo-2 fluoro-4 phényl) benzènecarbohy drazonoyle peut être préparé de la manière suivante : on opère comme à l'exemple 1 pour la préparation du bromure de N-(dibromo-2,4 phényl) benzènecarbohydrazonoyle, à partir de 20,2g de benzaldéhyde (fluoro-4 phényl) hydrazone et de 9,7 cm3 de brome dans 400 cm3 d'acide acétique. Le mélange est agité 30 minutes à reflux puis refroidi à une température proche de 20 °C. Après traitement habituel, on obtient 31,3g de bromure de N-(bromo-2 fluoro-4 phényl) benzènecarbohydrazonoyle fondant à 76 °C et utilisé à l'état brut dans les synthèses ultérieures.

La benzaldéhyde (fluoro-4 phényl) hydrazone peut être préparée selon la méthode décrite par B.A.

DELLACOLETTA et coll., J. Org. Chem., 3057(1977).

EXEMPLE 9

On opère comme à l'exemple 2, à partir de 2,1g d'acétyl-1 méthyl-6 phényl-3 1H-benzoxadiazine-4,1,2 et de 21 cm3 d'hydrazine hydrate. Le mélange est agité pendant 1 heure à une température proche de 70 °C puis refroidi à une température voisine de 20 °C. Après purification par recristallisation dans 19 cm3 d'éther de pétrole bouillant, on obtient 1,1g de méthyl-6 phényl-3 1H-benzoxadiazine-4,1,2 fondant à 85 °C.

L'acétyl-1 méthyl-6 phényl-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 32,5g d'acétyl-1 (bromo-2 méthyl-4 phényl)-1 benzoyl-2 hydrazine, de 47 cm3 de triéthylamine et de 9g de potasse en pastilles dans 83 cm3 de diméthylformamide. La solution est agitée pendant 4 heures à reflux puis refroidie à une température proche de 20 °C. Après purification par flash-chromatographie sur colonne de silice, sous courant d'argon, à moyenne pression (0,5-1,5 bar) avec un mélange d'acétate d'éthyle et de cyclohexane (30-70 en volumes) comme éluant et recristallisation dans 20 cm3 de cyclohexane bouillant, on obtient 2,1g d'acétyl-1 méthyl-6 phényl-3 1H-benzoxadiazine-4,1,2 fondant à 120 °C, utilisée à l'état brut dans les synthèses ultérieures.

L'acétyl-1 (bromo-2 méthyl-4 phényl)-1 benzoyl-2 hydrazine peut être préparée de la manière suivante : on opère comme à l'exemple 1 pour la préparation de l'acétyl-1 (dibromo-2,4 phényl)-1 benzoyl-2 hydrazine, à partir de 41g de bromure de N-(bromo-2 méthyl-4 phényl) benzènecarbohydrazonoyle et de 45,7g d'acétate de sodium dans 450 cm3 d'acide acétique. Le mélange est agité pendant 4 heures à reflux puis refroidi à une température proche de 20 °C. Après purification par recristallisation dans 250 cm3 d'éther de diisopropyle bouillant, on obtient 32,5g d'acétyl-1 (bromo-2 méthyl-4 phényl)-1 benzoyl-2 hydrazine fondant à 132 °C et utilisée à l'état brut dans les synthèses ultérieures.

Le bromure de N-(bromo-2 méthyl-4 phényl) benzénecarbohydrazonoyle peut être préparé de la manière suivante : on opère comme à l'exemple 1 pour la préparation du bromure de N-(dibromo-2,4 phényl) benzènecarbohydrazonoyle, à partir de 27,3g de benzaldéhyde (méthyl-4 phényl) hydrazone et de 13,4 cm3 de brome dans 300 cm3 d'acide acétique. Le mélange est agité 1 heure à une température proche de 20 °C. Après traitement habituel, on obtient 41g de bromure de N-(bromo-2 méthyl-4 phényl) benzenecarbohydrazonoyle fondant à 67 °C et utilisé à l'état brut dans les synthèses ultérieures.

La benzaldéhyde (méthyl-4 phényl) hydrazone peut être preparée selon la méthode décrite par B.A. DELLACOLETTA et coll., J. Org. Chem., 3057(1977).

EXEMPLE 10

On opère comme à l'exemple 2, à partir de 3,2g d'acétyl-1 isopropyl-6 phényl-3 1H-benzoxadiazine-4,1,2 et de 31,5 cm3 d'hydrazine hydrate. Le mélange est agité pendant 2 heures à une température proche de 70 °C puis refroidi à une température voisine de 20 °C. Après purification par flash-chromatographie sur colonne de silice, sous courant d'argon, à moyenne pression (0,5-1,5 bar) avec un mélange de dichlorométhane et de cyclohexane (80-20 en volumes) comme éluant et recristallisation dans 13 cm3 d'éther de pétrole bouillant, on obtient 1,9g d'isopropyl-6 phényl-3 1H-benzoxadiazine-4,1,2 fondant à 56 °C.

L'acétyl-1 isopropyl-6 phényl-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 25,5g d'acétyl-1 (bromo-2 isopropyl-4 phényl)-1 benzoyl-2 hydrazine, de 34 cm3 de triéthylamine et de 6,5g de potasse en pastilles dans 250 cm3 de diméthylformamide. La solution est agitée pendant 5 heures à reflux puis refroidie à une température proche de 20 °C. Après purification par flash-chromatographie sur colonne de silice, sous courant d'argon, à moyenne pression (0,5-1,5 bar) avec un mélange d'acétate d'éthyle et de cyclohexane (30-70 en volumes) comme éluant, on obtient 3,2g d'acétyl-1 isopropyl-6 phényl-3 1H-benzoxadiazine-4,1,2 fondant à 80 °C, utilisée à l'état brut dans les synthèses ultérieures.

L'acétyl-1 (bromo-2 fluoro-4 phényl)-1 benzoyl-2 hydrazine peut être préparée de la manière suivante : on opère comme à l'exemple 1 pour la préparation de l'acétyl-1 (dibromo-2,4 phényl)-1 benzoyl-2 hydrazine, à partir de 37 g de bromure de N-(bromo-2 isopropyl-4 phényl) benzènecarbohydrazonoyle et de 38,3g d'acétate de sodium dans 370 cm3 d'acide acétique. Le mélange est agité pendant 4 heures à reflux puis refroidi à une température proche de 20 °C. Après purification par recristallisation dans 190 cm3 d'éther de diisopropyle bouillant, on obtient 25,5 g d'acétyl-1 (bromo-2 isopropyl-4 phényl)-1 benzoyl-2

hydrazine fondant à 132 °C et utilisée à l'état brut dans les synthèses ultérieures.

Le bromure de N-(bromo-2 isopropyl-4 phényl) benzènecarbohydrazonoyle peut être préparé de la manière suivante : on opère comme à l'exemple 1 pour la préparation du bromure de N-(dibromo-2,4 phényl) benzenecarbohydrazonoyle, à partir de 22,4g de benzaldéhyde (isopropyl-4 phényl) hydrazone et de 9,7 cm3 de brome dans 220 cm3 d'acide acétique. Le mélange est agité 1 heure à une température proche de 20 °C. Après traitement habituel, on obtient 37g de bromure de N-(bromo-2 isopropyl-4 phényl) benzènecarbohydrazonoyle sous forme d'une huile rouge et utilisé à l'état brut dans les synthèses ultérieures.

La benzaldéhyde (isopropyl-4 phényl) hydrazone peut être préparée selon la méthode suivante : à 22,5g d'isopropyl-4 phénylhydrazine dans 150 cm3 de dichlorométhane, on coule en 15 minutes 15,3 cm3 de benzaldéhyde. La solution est agitée 1 heure à une température voisine de 25 °C puis séchée sur du sulfate de sodium anhydre, filtrée et concentrée à sec à 40 °C sous pression réduite (20 mm de mercure ; 2,7 kPa). Le résidu huileux est purifié par recristallisation dans 100 cm3 d'éther de diisopropyle bouillant. On obtient 27,2g de benzaldéhyde (isopropyl-4 phényl) hydrazone fondant à 120 °C, utilisée à l'état brut dans les synthèses ultérieures.

## EXEMPLE 11

On opère comme à l'exemple 2, à partir de 2,3g d'acétyl-1 iodo-6 phényl-3 1H-benzoxadiazine-4,1,2 et de 23 cm3 d'hydrazine hydrate. Le mélange est agité pendant 72 heures à une température proche de 50 °C puis refroidi à une température voisine de 20 °C. Après purification par recristallisation dans 70 cm3 de cyclohexane bouillant, on obtient 1,5g de iodo-6 phényl-3 1H-benzoxadiazine-4,1,2 fondant à 160 °C.

L'acétyl-1 iodo-6 phényl-3 1H-benzoxadiazine-4,1,2 peut être préparée de la manière suivante : à une suspension de 5,3g d'acétyl-1 amino-6 phényl-3 1H-benzoxadiazine-4,1,2 dans 24 cm3 d'eau distillée, on coule, à une température proche de 20 °C, 7,6 cm3 d'acide sulfurique 36N. Le milieu réactionnel est refroidi à une température voisine de 5 °C puis on ajoute 1,4g de nitrite de sodium dans 4 cm3 d'eau distillée. L'agitation est maintenue 1 heure à cette température, puis on additionne 4 g d'iodure de potassium dans 3 cm3 d'eau distillée. Après agitation 15 heures à une température proche de 20 °C et cessation du dégagement gazeux, le mélange est repris par 200 cm3 d'eau distillée ; la phase organique est extraite par 3 fois 150 cm3 de dichlorométhane, séchée sur du sulfate de sodium anhydre, filtrée et concentrée à sec à 40 °C sous pression réduite (20 mm de mercure ; 2,7 kPa). L'huile résiduelle est purifiée par flash-chromatographie sur colonne de silice, sous courant d'argon, à moyenne pression (0,5-1,5 bar) avec du dichlorométhane comme éluant. On obtient 2,3g d'acétyl-1 iodo-6 phényl-3 1H-benzoxadiazine-4,1,2 fondant à 138 °C, utilisée à l'état brut dans les synthèses ultérieures.

L'acétyl-1 amino-6 phényl-3 1H-benzoxadiazine-4,1,2 peut être préparée de la manière suivante : 217g d'acétyl-1 nitro-6 phé nyl-3 1H-benzoxadiazine-4,1,2, 105 cm3 d'acide chlorhydrique 12N et 10 cm3 d'éthanol sont portés à ébullition en agitant. On ajoute ensuite 123g de fer par petites portions dans le milieu réactionnel et maintient le reflux 4 heures et 30 minutes puis refroidi à une température proche de 20 °C. La suspension est filtrée sur célite et verre fritté, lavée par 3 fois 700 cm3 de dichlorométhane. Le filtrat est concentré à sec à 40 °C sous pression réduite (20 mm de mercure ; 2,7 kPa). Le résidu pâteux est repris par 5000 cm3 de dichlorométhane et 3000 cm3 d'eau distillée. La phase organique est décantée, traitée par 5g de noir végétal, séchée sur du sulfate de magnésium anhydre, filtrée et évaporée à sec à 40 °C sous pression réduite (20 mm de mercure ; 2,7 kPa). Le résidu est cristallisé dans 1000 cm3 d'éther de diisopropyle. On obtient 140g d'un solide noirâtre qui est purifié par flash-chromatographie sur colonne de silice, sous courant d'argon, à moyenne pression (0,5-1,5 bar) avec un mélange d'acétate d'éthyle et de cyclohexane (50-50 en volumes) comme éluant. On obtient 64,5g d'acétyl-1 amino-6 phényl-3 1H-benzoxadiazine-4,1,2 fondant à 140 °C et utilisée à l'état brut dans les synthèses ultérieures.

L'acétyl-1 nitro-6 phényl-3 1H-benzoxadiazine-4,1,2 peut être préparée de la façon suivante : on opère comme à l'exemple 2 pour la préparation de l'acétyl-1 (pyridyl-3)-3 1H-benzoxadiazine-4,1,2, à partir de 755g d'acétyl-1 (bromo-2 nitro-4 phényl)-1 benzoyl-2 hydrazine, de 1000 cm3 de triéthylamine et de 80g de soude en pastilles dans 5000 cm3 de diméthylformamide. La solution est agitée pendant 2 heures à reflux puis refroidie à une température proche de 20 °C. Après purification par recristallisation dans 6000 cm3 d'acétate d'éthyle bouillant, on obtient 196g d'acétyl-1 nitro-6 phényl-3 1H-benzoxadiazine-4,1,2 fondant à 201 °C, utilisée à l'état brut dans les synthèses ultérieures.

L'acétyl-1 (bromo-2 nitro-4 phényl)-1 benzoyl-2 hydrazine peut être préparée de la manière suivante : on opère comme à l'exemple 1 pour la préparation de l'acétyl-1 (dibromo-2,4 phényl)-1 benzoyl-2 hydrazine, à partir de 1015g de bromure de N-(bromo-2 nitro-4 phényl) benzènecarbohydrazonoyle et de

1037g d'acétate de sodium dans 25 cm3 d'acide acétique. Le mélange est agité pendant 4 heures à reflux puis refroidi à une température proche de 20°C. Après traitement habituel, on obtient 755g d'acétyl-1 (bromo-2 nitro-4 phényl)-1 benzoyl-2 hydrazine sous forme d'une meringue et utilisée à l'état brut dans les synthèses ultérieures.

Le bromure de N-(bromo-2 nitro-4 phényl) benzènecarbohydrazonoyle peut être préparé de la manière suivante : on opère comme à l'exemple 1 pour la préparation du bromure de N-(dibromo-2,4 phényl) benzènecarbohydrazonoyle, à partir de 733g de benzaldéhyde (nitro-4 phényl) hydrazone et de 1343 cm3 de brome dans 13,5 cm3 d'acide acétique. Le mélange est agité 2 heures 30 minutes à reflux puis refroidi à une température proche de 20°C. Après traitement habituel, on obtient 1015g de bromure de N-(bromo-2 nitro-4 phényl) benzénecarbohydrazonoyle fondant à 173°C et utilisé à l'état brut dans les synthèses ultérieures.

La benzaldéhyde (nitro-4 phényl) hydrazone peut être préparée selon la méthode décrite par H. NAGANO et coll., Chem. Pharm. Bull., 35(10), 4093(1987).

EXEMPLE 12

3,4g d'acétyl-1 (fluoro-2 phényl)-1 (méthoxy-2 benzoyl)-2 hydrazine, 0,5g de soude en pastilles broyées et 6 cm3 de triéthylamine dans 30 cm3 de diméthylformamide sont agités et chauffés à ébullition pendant 6 heures. Le mélange est concentré à 80°C sous pression réduite (20 mm de mercure, 2,7 kPa). L'huile résiduelle est reprise par 100 cm3 de dichlorométhane, la phase organique lavée par 2 fois 50 cm3 d'eau, décantée, séchée sur du sulfate de sodium anhydre, filtrée et concentrée à sec à 40°C sous pression réduite (20 mm de mercure ; 2,7 kPa). Le résidu est agité et chauffé à 60°C avec 60 cm3 d'hydrazine hydrate pendant 6 heures. Après refroidissement à une température voisine de 20°C on ajoute 200 cm3 d'eau et la phase organique est extraite par 2 fois avec 50 cm3 de dichlorométhane, décantée, séchée sur du sulfate de sodium anhydre, filtrée et concentrée à sec à 40°C sous pression réduite (20 mm de mercure 2,7 kPa). L'huile résiduelle est chromatographiée sur colonne de silice avec un mélange d'acétate d'éthyle et d'hexane (30-70 en volumes) comme éluant. On obtient après recristallisation dans le méthanol bouillant 0,25g de (méthoxy-2 phényl)-3 1H benzoxadiazine-4,1,2 fondant à 140°C.

L'acétyl-1 (fluoro-2 phényl)-1 (méthoxy-2 benzoyl)-2 hydrazine peut être préparée de la façon suivante : à 4,8g de (fluoro-2 phényl)-1 (méthoxy-2 benzoyl)-2 hydrazine et 1,8 cm3 de pyridine dans 100 cm3 de dichlorométhane refroidis à 0°C on ajoute goutte à goutte 1,3g de chlorure d'acétyle dans 20 cm3 de dichlorométhane. Le mélange est agité 1 heure à une température voisine de 20°C puis est repris par 100 cm3 d'eau. La phase organique est décantée, lavée avec 20 cm3 d'une solution d'acide chlorhydrique 1N puis 50 cm3 d'eau, décantée séchée sur du sulfate de sodium anhydre, filtrée et concentrée à sec. L'huile résiduelle est chromatographiée sur colonne de silice avec un mélange d'acétate d'éthyle et d'hexane (50-50 en volumes) comme éluant. Le produit isolé (5,2g) est recristallisé dans l'acétate d'éthyle bouillant. On obtient 3,7g d'acétyl-1 (fluoro-2 phényl)-1 (méthoxy-2 benzoyl)-2 hydrazine fondant à 124°C.

La (fluoro-2 phényl)-1 (méthoxy-2 benzoyl)-2 hydrazine peut être préparée de la manière suivante : à 4,95g de chlorhydrate d'orthofluorophénylhydrazine et 7 cm3 de pyridine dans 50 cm3 de dichlorométhane anhydre refroidi à 0°C on ajoute, goutte à goutte, 5,3g de chlorure d'orthométhoxybenzoyle dans 20 cm3 de dichlorométhane. L'agitation est maintenue 1 heure à une température voisine de 20°C. Après traitement habituel le résidu est chromatographié sur colonne de silice (200g) avec un mélange d'acétate d'éthyle et d'hexane (30-70 puis 40-60 en volumes) comme éluant. Après recristallisation dans le méthanol bouillant, on obtient 4,85g de (fluoro-2 phényl)-1 (méthoxy-2 benzoyl)-2 hydrazine fondant à 83°C.

EXEMPLE 13

On opère comme à l'exemple 12, à partir de 3,8g d'acétyl-1 (fluoro-2 phényl)-1 (méthoxy-3 benzoyl)-2 hydrazine, de 0,55g de soude en pastilles broyées et de 6 cm3 de triéthylamine dans 30 cm3 de diméthylformamide. Le mélange est agité et chauffé 6 heures à ébullition. Après extraction au dichlorométhane le produit isolé est ensuite traité par 40 cm3 d'hydrazine hydrate à 60°C pendant 6 heures. Après traitement habituel, le produit est chromatographié sur une colonne de silice avec un mélange de dichlorométhane et d'hexane (50-50 en volumes) puis du dichlorométhane pur comme éluants. Après recristallisation dans un mélange de méthanol et d'éther diéthylique bouillant (10-90 en volumes) on obtient 0,60g de (méthoxy-3 phényl)-3 1H-benzoxadiazine-4,1,2 fondant à 87°C.

L'acétyl-1 (fluoro-2 phényl)-1 (méthoxy-3 benzoyl)-2 hydrazine peut être préparée de la manière

suivante : on opère comme à l'exemple 12 pour la préparation de l'acétyl-1 (fluoro-2 phényl)-1 (méthoxy-2 benzoyl)-2 hydrazine à partir de 3,9g de (fluoro-2 phényl)-1 (méthoxy-3 benzoyl)-2 hydrazine, de 1,4 cm3 de pyridine dans 50 cm3 de dichlorométhane anhydre refroidi à 0° C et de 1,1 cm3 de chlorure d'acétyle dans 20 cm3 de dichlorométhane. Le mélange est agité 1 heure à une température voisine de 20° C. Après traitement habituel on obtient une huile (4,3g) constituée d'un mélange d'acétyl-1 (fluoro-2 phényl)-1 (méthoxy-3 benzoyl)-2 hydrazine et d'acétyl-2 (fluoro-2 phényl)-1 (méthoxy-3 benzoyl)-2 hydrazine (70-30 en poids). Par recristallisation de cette huile dans un mélange d'éther diéthylique et de pentane bouillant on obtient 3g d'acétyl-1 (fluoro-2 phényl)-1 (méthoxy-3 benzoyl) hydrazine pure fondant à 134° C.

La (fluoro-2 phényl)-1 (méthoxy-2 benzoyl)-2 hydrazine peut être préparée de la manière suivante : on opère comme à l'exemple 12 pour la préparation de la (fluoro-2 phényl)-1 (méthoxy-3 benzoyl)-2 hydrazine à partir de 5,4g d'orthofluorophénylhydrazine, de 3,5 cm3 de pyridine dans 50 cm3 de dichlorométhane anhydre refroidi à -10° C et de 6,8g de chlorure de méthoxy-2 benzoyle en solution dans 30 cm3 de dichlorométhane. L'agitation est maintenue 1 heure à une température voisine de 20° C. Après traitement habituel, le produit est isolé et recristallisé dans un mélange de dichlorométhane et de pentane bouillant ; on obtient 7g de (fluoro-2 phényl)-1 (méthoxy-2 benzoyl)-2 hydrazine fondant à 122° C.

EXEMPLE 14

A une solution agitée de 0,07g de bromure de N-(hydroxy-2 phényl) benzènecarbohydrazonoyle dans 1 cm3 de méthanol anhydre on ajoute, goutte à goutte, à -50° C, 2,5 cm3 d'une solution 0,1M de méthylate de sodium dans le méthanol. Après refroidissement à une température voisine de 20° C et évaporation du méthanol sous pression réduite (20 mm de mercure ; 2,7 kPa) le produit est chromatographié sur plaque de silice avec un mélange d'acétate d'éthyle et d'hexane (30-70 en volumes) comme éluant. Le produit est recristallisé dans un mélange de méthanol et de dichlorométhane bouillant, on obtient 0,03g de phényl-3 1H-benzoxadiazine-4,1,2 fondant à 98° C.

Le bromure de N-(hydroxy-2 phényl) benzènecarbohydrazonoyle peut être préparé de la façon suivante : à 0,61g de bromure de N-(méthoxy-2 phényl) benzènecarbohydrazonoyle en solution dans 8 cm3 de dichlorométhane sont ajoutés à -10° C avec agitation, goutte à goutte, 0,2 cm3 de tribromure de bore. L'agitation est maintenue 1 heure à 0° C. Après hydrolyse avec 10 cm3 d'un mélange de glace et d'eau, la phase organique est lavée à l'eau, séchée sur du sulfate de sodium anhydre, filtrée et évaporée sous pression réduite (20 mm de mercure ; 2,7 kPa). Après recristallisation dans un mélange d'éther diéthylique et de pentane bouillant, on obtient 0,35g de bromure de N-(hydroxy-2 phényl) benzènecarbohydrazonoyle fondant à 120° C.

Le bromure de N-(méthoxy-2 phényl) benzènecarbohydrazonoyle peut être préparé de la façon suivante : à 0,97g de (méthoxy-2 phényl)-1 benzoyl-2 hydrazine dans 8 cm3 d'acétonitrile anhydre, agités à une température voisine de 20° C on ajoute, à la spatule, successivement 1,33g de tétrabromure de carbone et 1,3g de triphénylphosphine. L'agitation est maintenue sous argon pendant 15 heures. Après addition de 50 cm3 d'eau et extraction par 2 fois 15 cm3 d'éther diéthylique, les phases organiques sont lavées par 3 fois 30 cm3 d'eau, décantées, séchées sur du sulfate de sodium anhydre et filtrées. Après évaporation de l'éther, le produit cristallisé est chromatographié sur un colonne de silice avec un mélange d'acétate d'éthyle et d'hexane (30-70 en volumes) comme éluant et recristallisé dans un mélange de méthanol et de dichlorométhane. On obtient 0,96g de bromure N-(méthoxy-2 phényl) benzènecarbohydrazonoyle fondant à 113° C.

La (méthoxy-2 phényl)-1 benzoyl-2 hydrazine peut être préparée de la façon suivante : à une solution de 1,22g de méthoxy-2 phénylhydrazine et de 1,1 cm3 de pyridine dans 15 cm3 d'éther diéthylique anhydre, on ajoute goutte à goutte à une température de 0° C, une solution de 1,6g de chlorure de benzoyle dans 5 cm3 d'éther diéthylique, l'agitation est maintenue 2 heures à une température proche de 20° C. Le mélange est repris par 30 cm3 d'une solution d'acide chlohydrique 1N puis avec 30 cm3 d'eau, la phase organique est décantée, séchée sur du sulfate de sodium anhydre, filtrée et évaporée à sec. Après recristallisation du produit dans un mélange d'éther diéthylique et de pentane bouillant on obtient 2g de (méthoxy-2 phényl)-1 benzoyl-2 hydrazine fondant à 148° C.

EXEMPLE 15

A une solution de 0,20g de (méthoxy-3 phényl)-3 1H benzoxadiazine-4,1,2 dans 2 cm3 de dichlorométhane refroidie à 0° C on ajoute, goutte à goutte, 0,20 cm3 de tribromure de bore puis on agite pendant 4

heures à une température voisine de 20 ° C. Le mélange est repris par 10 cm3 d'eau et de glace et extrait par 2 fois 5 cm3 de dichlorométhane ; la phase organique est décantée, lavée par 2 fois 10 cm3 d'une solution aqueuse de bicarbonate de sodium à 5% puis par 2 fois 10 cm3 d'eau, séchée sur du sulfate de sodium anhydre, filtrée et concentrée à sec à 40 ° C sous pression réduite (20 mm de mercure, 2,7 kPa). Par recristallisation dans un mélange de dichlorométhane et d'hexane bouillant, on obtient 0,08g d'(hydroxy-3 phényl)-3 1H-benzoxadiazine-4,1,2.

EXEMPLE 16

On opère comme à l'exemple 15 à partir d'une solution de 0,14g de (méthoxy-2 phényl)-3 1H benzoxadiazine-4,1,2 dans 2 cm3 de dichlorométhane refroidie à 0 ° C et de 1,4 cm3 d'une solution 1M de tribromure de bore dans le dichlorométhane. L'agitation est maintenue pendant 1 heure à une température proche de 20 ° C. Le mélange est repris par 10 cm3 d'eau et de glace. Après traitement habituel le produit cristallisé est filtré sur une colonne de silice avec du dichlorométhane comme éluant et recristallisé dans un mélange de dichlorométhane et d'hexane bouillant. On obtient 0,12g d'(hydroxy-2 phényl)-3 1H-benzoxadiazine-4,1,2 fondant à 154 ° C.

EXEMPLE 17

A 0,27g d'orthoisopropylaminophényl benzoylhydrazide dans 3 cm3 d'acétonitrile anhydre sont ajoutés successivement à une température voisine de 20 ° C, avec agitation, 0,34g de tétrabromure de carbone et 0,33g de triphénylphosphine. L'agitation est maintenue 8 heures. Après addition de 20 cm3 d'eau on extrait au dichlorométhane. La phase organique est lavée à l'eau, décantée, séchée sur du sulfate de sodium anhydre et concentrée. Après chromatographie du résidu sur colonne de silice avec un mélange d'acétate d'éthyle et d'hexane (20-80 en volumes) comme éluant puis recristallisation dans du méthanol bouillant, on obtient 0,10g d'isopropyl-4 phényl-3 1H-benzotriazine-1,2,4 fondant à 250 ° C.

L'orthoisopropylaminophényl benzoylhydrazide peut être préparé de la façon suivante : une solution de 1,13g d'orthoaminophényl benzoylhydrazide dans 7 cm3 d'hexaméthylphosphortriamide est agitée avec 2g d'hydrogénocarbonate de sodium anhydre et 1 cm3 d'iodure d'isopropyle, sous argon, pendant 15 heures à une température voisine de 20 ° C. Après addition de 50 cm3 d'eau, on extrait par 2 fois 30 cm3 de dichlorométhane. Les fractions organiques sont lavées 3 fois à l'eau, décantées, séchées sur du sulfate de sodium anhydre et concentrées. Le produit brut est filtré sur une colonne de silice avec du dichlorométhane comme éluant et recristallisé dans un mélange d'acétate d'éthyle et d'hexane. On obtient 1,05g d'orthoisopropylaminophényl benzoylhydrazide utilisé à l'état brut dans les syntèses ultérieures.

L'orthoaminophényl benzoylhydrazide peut être préparé selon la méthode décrite par M.M. SHENG, J. Org. Chem., 28, 736(1963).

EXEMPLE 18

On opère comme à l'exemple 14, à partir de 2,4g de bromure de N-(méthoxy-2 bromo-4 phényl) pentafluoro-2,3,4,5,6 benzènecarbohydrazonoyle dans 60 cm3 de dichlorométhane anhydre auxquels on ajoute, goutte à goutte, à une température voisine de 20 ° C, 1 cm3 de tribromure de bore. Le mélange est agité 5 heures. Après traitement habituel l'huile obtenue est dissoute dans 30 cm3 de méthanol anhydre et on ajoute goutte à goutte, en agitant, à -50 ° C 25 cm3 d'une solution, 0,2M de méthylate de sodium dans le méthanol. La solution est concentrée à sec à 40 ° C sous pression réduite (20 mm de mercure ; 2,7 kPa) et le résidu est chromatographié sur colonne de silice avec un mélange d'acétone et d'hexane (30-70 en volumes) comme éluant. Après recristallisation dans un mélange de méthanol et de dichlorométhane on obtient 0,54g de bromo-6 (pentafluoro-2,3,4, 5,6 phényl)-3 1H-benzoxadiazine-4,1,2 fondant à 184 ° C.

Le bromure de N-(méthoxy-2 bromo-4 phényl) pentafluoro-2,3,4,5,6 benzènecarbohydrazonoyle peut être préparé de la façon suivante : à une solution de 7,7g de pentafluoro-2,3,4,5,6 benzaldéhyde méthoxy-2 phénylhydrazone dans 150 cm3 de tétrahydrofuranne anhydre sont ajoutés à 0 ° C, par petites fractions, avec agitation, 21g de tribromure de phényltriméthylammonium. On agite 3 heures à une température voisine de 20 ° C et la solution est versée dans 500 cm3 d'eau et extraite par 2 fois 150 cm3 d'éther diéthylique. La phase organique est lavée 2 fois à l'eau, décantée, séchée sur du sulfate de sodium anhydre et concentrée à sec. Après recristallisation dans un mélange de méthanol et de dichlorométhane

on obtient 10g de bromure de N-(méthoxy-2 bromo-4 phényl) pentafluoro-2,3,4,5,6 benzènecarbohydrazonoyle fondant à 104° C.

La pentafluoro-2,3,4,5,6 benzaldéhyde méthoxy-2 phénylhydrazone peut être préparée de la façon suivante : à une solution de 5,7g d'orthométhoxyphénylhydrazine dans 30 cm3 de méthanol on ajoute goutte à goutte, avec agitation, une solution de 5,88 g de pentafluoro-2,3,4,5,6 benzaldéhyde en solution dans 20 cm3 de méthanol. On agite pendant 5 heures et filtre le précipité. Après recristallisation dans un mélange de dichlorométhane et de méthanol on obtient 8,5g de pentafluoro-2,3,4,5,6 benzaldéhyde méthoxy-2 phenylhydrazone fondant à 204° C.

EXEMPLE 19

1,5g d'acétyl-1 bromo-6 phényl-3 1H-benzothiadiazine-4,1,2 dans 60 cm3 d'éthanol et 40 cm3 d'acide chlorhydrique concentré (d = 1,18) sont chauffés à ébullition pendant 4 heures puis le mélange est refroidi à une température voisine de 20° C. Après filtration et recristallisation dans l'éthanol bouillant, on obtient 0,58g de bromo-6 phényl-3 1H-benzothiadiazine-4,1,2 qui fond à 122° C.

L'acétyl-1 bromo-6 phényl-3 1H-benzothiadiazine-4,1,2 peut être préparé de la façon suivante : 4,33g de bromure de N-(dibromo-2,4 phényl) benzénecarbohydrazonoyle et 2,28g de thioacétate de potassium dans 100 cm3 d'acétonitrile sont chauffés à ébullition pendant 4 heures. Après refroidissement à une température voisine de 20° C, on ajoute 300 cm3 d'eau et extrait par 2 fois 200 cm3 de dichlorométhane. La fraction organique est lavée par 3 fois 100 cm3 d'eau, décantée, séchée sur sulfate de sodium anhydre et évaporée à sec. Le résidu est chromatographié sur colonne de silice avec un mélange d'acétate d'éthyle et d'hexane (30-70 en volumes) comme éluant. Après recristallisation dans l'acétonitrile bouillant, on obtient 0,48g de bromo-6 phényl-3 1H-benzothiadiazine-4,1,2 fondant à 182° C.

Les médicaments selon l'invention sont constitués par les composés de formule (I) à l'état pur ou sous forme d'une composition dans laquelle il est associé à tout autre produit pharmaceutiquement compatible, pouvant être inerte ou physiologiquement actif. Ces médicaments peuvent être utilisés par voie topique.

Les compositions pour amdinistration topique peuvent être par exemple des crèmes, pommades, lotions, collyres, collutoires, nébulisations ou pulvérisations.

Les compositions stériles pour administration locale (nébulisations ou pulvérisations) peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple, par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile.

En thérapeutique humaine, les médicaments selon l'invention sont particulièrement utiles comme antiinflammatoires, pour le traitement de l'asthme, des maladies allergiques, du psoriasis ainsi que pour le traitement de tout phénomène pathologique cutané lié à l'hyperactivité de la 5-lipoxygènase et dela cyclooxygènase.

Les doses dépendent de l'effet recherché, de la durée du traitement et de la voie d'administration utilisée ; elles sont généralement comprises entre 0,05 et 1g par jour par voie topique pour un adulte avec des doses unitaires allant de 10 à 500 mg de substance active.

D'une façon générale, le médecin déterminera la posologie appropriée en fonction de l'âge, du poids et de tous les autres facteurs propres au sujet à traiter.

L'exemple suivant illustre une composition selon l'invention :

| | |
|---|---|
| - (pyridyl-3)-3 1H-benzoxadiazine-4,1,2 ..... | 100 mg |
| - vaseline ...... | qsp 20 g |

**Revendications**

1 - Médicaments caractérisés en ce qu'ils contiennent en tant que principe actif au moins un composé de formule :

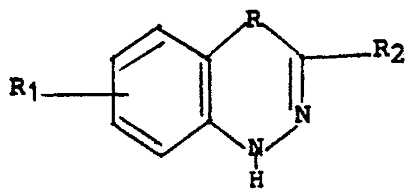

(I)

dans laquelle,

- R représente un atome d'oxygène, de soufre ou d'azote substitué par un radical alkyle,
- $R_1$ représente un atome d'hydrogène ou d'halogène ou un radical alkyle,
- $R_2$ représente un radical phényle, phényle substitué (par un radical alcoxy ou hydroxy), pyridyle, thiényle, naphtyle ou pentafluoro-2,3,4,5,6 phényle, étant entendu que les radicaux alkyle et alcoxy contiennent 1 à 4 atomes de carbone en chaîne droite ou ramifiée.

2 - Composés de formule :

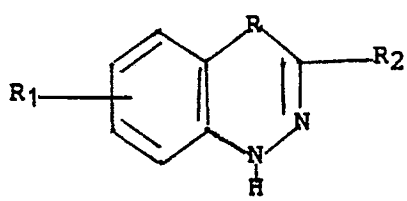

(I)

dans laquelle,

- R représente un atome d'oxygène, de soufre ou d'azote substitué par un radical alkyle,
- $R_1$ représente un atome d'hydrogène ou d'halogène ou un radical alkyle,
- $R_2$ représente un radical phényle, phényle substitué (par un radical alcoxy ou hydroxy), pyridyle, thiényle, naphtyle ou pentafluoro-2,3,4,5,6 phényle, à l'exception de la bromo-6 phényl-3 1H-benzoxadiazine-4,1,2 les bromo-6, -7 ou -8 ou fluoro-6 phényl-3 1H-benzothiadiazine-4,1,2 la bromo-6 p-méthoxyphényl-3 1H-benzothiadiazine-4,1,2 et la phényl-3 1H-benzothiadiazine-4,1,2, et étant entendu que les radicaux alkyle et alcoxy contiennent 1 à 4 atomes de carbone en chaîne droite ou ramifiée.

3 - Procédé de préparation des composés de formule (I) selon la revendication 2 pour lesquels R représente un atome d'oxygène ou de soufre caractérisé en ce que l'on désacétyle un dérivé de formule :

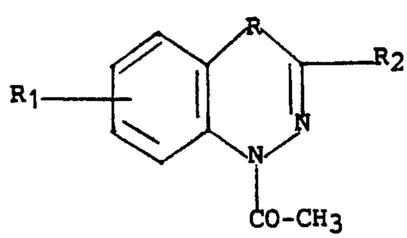

(II)

dans laquelle $R_1$ et $R_2$ ont les mêmes significations que dans la revendication 2 et R représente un atome d'oxygène ou de soufre puis isole le produit.

4 - Procédé de préparation des composés de formule (I) selon la revendication 2 pour lesquels R représente un atome d'oxygène, à l'exception de ceux pour lesquels $R_2$ représente un radical alcoxyphényle caractérisé en ce que l'on cyclise un dérivé de formule :

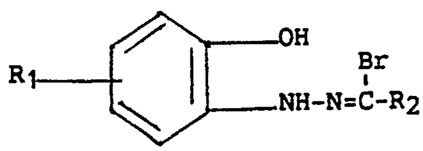

(XIV)

dans laquelle $R_1$ et $R_2$ ont les mêmes significations que dans la revendication 2 excepté que $R_2$ ne peut pas représenter un radical alcoxyphényle, puis isole le produit.

5 - Procédé de préparation des composés de formule (I) selon la revendication 2 pour lesquels R représente un atome d'azote substitué par un radical alkyle à l'exception de ceux pour lesquels $R_2$ représente un radical hydroxyphényle caractérisé en ce que l'on cyclise un dérivé de formule :

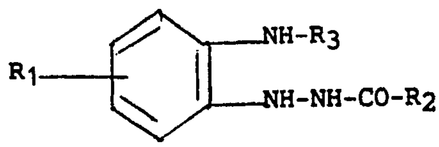

(XXI)

dans laquelle $R_1$ et $R_2$ ont les mêmes significations que dans la revendication 2 excepté que $R_2$ ne peut pas représenter un radical hydroxyphényle et $R_3$ représente un radical alkyle puis isole le produit.

6 - Procédé de préparation des composés de formule (I) selon la revendication 2 pour lesquels $R_2$ représente un radical hydroxyphényle caractérisé en ce que l'on déméthyle un dérivé de formule (I) correspondant pour lequel $R_2$ représente un radical alcoxyphényle puis isole de produit.

Revendications pour les Etats contractants suivants: ES, GR

1 - Procédé de préparation des composés de formule :

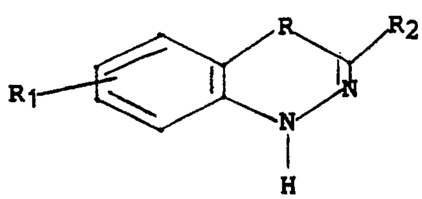

(I)

dans laquelle

- R représente un atome d'oxygène, de soufre ou d'azote substitué par un radical alkyle,
- $R_1$ représente un atome d'hydrogène ou d'halogène ou un radical alkyle
- $R_2$ représente un radical phényle, phényle substitué (par un radical alcoxy ou hydroxy), pyridyle, thiényle, naphtyle ou pentafluoro-2, 3, 4, 5, 6 phényle à l'exception de la bromo-6 phényl-3 1H-benzoxadiazine-4, 1, 2 , les bromo-6, -7 ou -8 ou fluoro-6 phényl-3 1H-benzothiadiazine -4, 1, 2, la bromo-6 p-méthoxyphényl-3 1H-benzothiadiazine-4, 1, 2 et la phényl-3 1H-benzothiadiazine-4, 1, 2, étant entendu que les radicaux alkyle et alcoxy contiennent 1 à 4 atomes de carbone en chaîne droite ou ramifiée caractérisé en ce que

A - pour la préparation des composés de formule (I) pour lesquels R représente un atome d'oxygène ou de soufre, on désacétyle un dérivé de formule :

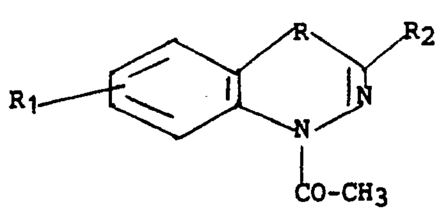

(II)

dans laquelle $R_1$ et $R_2$ ont les mêmes significations que dans la formule (I) et R représente un atome d'oxygène ou de soufre, puis on isole le produit,

B - pour la préparation des composés de formule (I) pour lesquels R représente un atome d'oxygène à l'exception de ceux pour lesquels $R_2$ représente un radical alcoxyphényle, on cyclise un dérivé de formule :

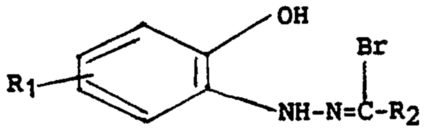

(XIV)

dans laquelle $R_1$ et $R_2$ ont les mêmes significations que dans la formule (I) excepté que $R_2$ n'est pas un radical alcoxyphényle, puis isole le produit

C - pour la préparation des composés de formule (I) pour lesquels R représente un atome d'azote substitué par un radical alkyle à l'exception de ceux pour lesquels $R_2$ représente un radical hydroxyphényle, on cyclise un dérivé de formule :

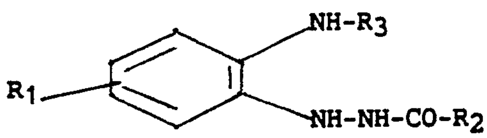

(XXI)

dans laquelle $R_1$ et $R_2$ ont les mêmes significations que la formule (I) excepté que $R_2$ ne peut pas représenter un radical hydroxyphényle et $R_3$ représente un radical alkyle, puis isole le produit,

D - pour la préparation des composés de formule (I) pour lesquels $R_2$ représente un radical hydroxyphényle, on déméthyle un dérivé de formule (I) correspondant pour lequel $R_2$ représente un radical alcoxyphényle puis isole le produit.

2 - Utilisation des composés de formule :

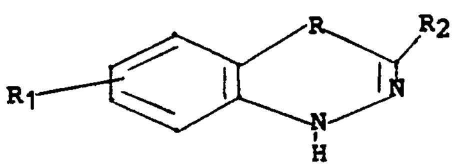

(I)

dans laquelle

- R représente un atome d'oxygène, de soufre ou d'azote substitué par un radical alkyle
- $R_1$ représente un atome d'hydrogène ou d'halogène ou un radical alkyle.
- $R_2$ représente un radical phényle, phényle substitué (par un radical alcoxy ou hydroxy), pyridyle, thiényle, naphtyle ou pentafluoro-2, 3, 4, 5, 6 phényle pour la préparation d'un médicament antiinflammatoire.

3 - Utilisation selon la revendication 2 pour la préparation d'un médicament pour le traitement de tout phénomène cutané lié à l'hyperactivité de la 5-lipoxygénase et de la cyclooxygénase.

4 - Utilisation selon la revendication 2 pour la préparation d'un médicament pour le traitement de l'asthme.

Office européen des brevets

# RAPPORT DE RECHERCHE EUROPEENNE

Numero de la demande

EP 90 40 1975

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
| --- | --- | --- | --- |
| A | FR-A-1 440 629 (SIEGFRIED)<br>* En entier *<br>----- | 1-6 | C 07 D 273/04<br>C 07 D 413/04<br>C 07 D 253/10<br>C 07 D 285/16<br>A 61 K 31/53<br>A 61 K 31/535<br>A 61 K 31/54 |

DOMAINES TECHNIQUES RECHERCHES (Int. Cl.5)

A 61 K 31/00
C 07 D 273/00
C 07 D 285/00
C 07 D 413/00
C 07 D 253/00
C 07 D 417/00
C 07 D 401/00
C 07 D 409/00

Le présent rapport a été établi pour toutes les revendications

| Lieu de la recherche | Date d'achèvement de la recherche | Examinateur |
| --- | --- | --- |
| LA HAYE | 02-10-1990 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)